# EUROPEAN PATENT APPLICATION

(11) **EP 1 457 553 A1**
(43) Date of publication of application: **15.09.2004**
(21) Application number: 02791976.0
(22) Date of filing: 19.12.2002
(51) Int. Cl.: C12N 15/00, C12M 1/00, C12Q 1/68, G01N 33/53

(54) **PLASTIC PLATE AND PLASTIC PLATE ASSEMBLY**

(30) Priority: 28.12.2001 JP 2001400562; 17.07.2002 JP 2002208114; 05.12.2002 JP 2002354361
(71) Applicant: ENPLAS CORPORATION, Kawaguchi-shi, Saitama-ken 332-0034 (JP)
(72) Inventor: WATANABE, Yasuhiro, Soka-shi, Saitama 340-0005 (JP); OHKUMA, Junichi, Koshigaya-shi, Saitama 343-0023 (JP)
(74) Representative: Manitz, Finsterwald & Partner GbR
(86) International application number: PCT/JP2002/013281
(87) International publication number: WO 2003/057873

(57) **Abstract**

There is provided a plastic plate and a plastic plate assembly, which can increase the amount of samples, such as gene fragments and specimens to be hybridized, to more precisely analyze genes. A plastic plate 2 is formed so as to be capable of housing a plurality of samples to be analyzed. A large number of wells (recessed portions) 3 for housing therein samples are formed in the surface 7 of the plastic plate 2 by transferring the shape of a metal pattern. Thus, a sufficient amount of samples can be housed in the wells 3, so that it is possible to efficiently and more precisely inspect, analyze and house therein various samples.

## Description

### Technical Field

The present invention relates generally to a plastic plate and a plastic plate assembly, which are used for analyzing samples, e.g., micro vital substances such as viruses and bacteria, vital formations such as cells and biopolymers, organic compounds other than biopolymers, inorganic substances and inorganic compounds. More specifically, the invention relates to a sample housing plastic plate and plastic plate assembly, which are used for simultaneously examining the functions of a large number of genes and which also particularly function as a reactor in the PCR method for amplifying DNAs.

### Background Art

In recent years, of various methods for examining the functions of genes, the microarray technique capable of simultaneously examining thousands kinds to tens of thousands kinds of genes has been particularly noticed in the field of genome medical science.

The microarray technique is a technique for examining the functions of genes bymeans of a microarray (which is also called DNA microchip) wherein thousands kinds to tens of thousands kinds of gene fragments are densely arrayed on a glass plate substantially having the same size as that of a human thumb. According to the microarray technique, if cDNAs (specimens) treated with a fluorescence dye are dropped onto a microarray, on which thousands kinds to tens of thousands kinds of gene fragments are applied, to hybridize the gene fragments on the microarray with the dropped cDNAs, only genes bonded to the cDNAs fluoresce, so that it is possible to identify active genes at a first sight.

For example, Japanese Patent Laid-Open No. 2000-235036 discloses a microarray technique wherein a spotter device is designed to apply gene fragments on the flat surface of a glass plate substantially having the same size as that of a human thumb.

However, in the conventional microarray disclosed in Japanese Patent Laid-OpenNo. 2000-235036, although the spotter device is designed to apply gene fragments on the flat surface of the glass plate as described above, it is required to densely spot thousands to tens of thousands gene fragments on the glass plate substantially having the same size as that of a human thumb, so that the amount of gene fragments to be applied is easily decreased to cause a variation in amount of gene fragments to be applied. Thus, it has been pointed out that there is the possibility that the results of the analysis of genes using the conventional microarray may include a lot of errors.

### Disclosure of the Invention

It is therefore an object of the present invention to eliminate the aforementioned problems and to provide a plastic plate and a plastic plate assembly, which can efficiently and more precisely inspect, analyze and house various samples.

It is another object of the present invention to provide a plastic plate and a plastic plate assembly, which can increase the amount of samples, such as gene fragments and specimens, to be hybridized, to more precisely analyze genes.

According to the present invention, there is provided a plastic plate capable of housing a plurality of samples to be analyzed, wherein a plurality of recessed portions for housing therein the samples are formed in a surface of the plastic plate by transferring a shape of a metal pattern. Furthermore, throughout the specification, the term "analysis" means to analyze samples to clarify facts as well as to examine, prepare, incubate, cultivate, synthesize, inspect, react or store samples.

A heater housing portion may be formed in the reverse of the plastic plate so as to correspond to the recessed portions. Alternatively, a heater housing portion may be formed in the reverse of the plastic plate so as to correspond to the recessed portions, and a reinforcing rib may be formed on the reverse of the plastic plate.

In the above described plastic plate, each of the recessed portions preferably has a sectional shape taken in depth directions thereof, the sectional shape being a trapezoid so that the opening portion of each of the recessed portions has a larger area than that of a bottom thereof. A large number of protrusions may be formed on a bottom of each of the recessed portions. The samples may contain DNA fragments. The plastic plate may be made of a resin containing a light absorbable material. A light absorbing coating may be formed on the surface of the plastic plate between adjacent two of the recessed portions. A light absorbable coating may be formed on the reverse of the plastic plate so as to separate the recessed portions from each other. Each of the recessed portions preferably has a depth which is smaller than a width thereof. Moreover, a groove may be formed in the surface of the plastic plate between adjacent two of the plurality of recessed portions. A groove surrounding each of the recessed portions may be formed in the surface of the plastic plate. The opening edge of each of the recessed portions may be formed so as to protrude from the surface of the plastic plate. A sample absorbing layer surrounding the opening edge of each of the recessed portions may be formed on the surface of the plastic plate.

According to the present invention, a plastic plate assembly comprises: a plurality of plastic plates capable of housing a plurality of samples to be analyzed; and a frame having a plurality of plate holding holes for housing and holding therein the plurality of plastic plates, wherein a plurality of recessed portions for housing therein the samples are formed in a surface of each of the plastic plates by transferring a shape of a metal pattern.

In this plastic plate assembly, two pairs of side faces of each of the plastic plates are preferably supported on side faces of a corresponding one of the plate holding holes at three points, respectively.

In the above described plastic plate assembly, each of the plastic plates may have a flange portion which has a bottom surface supported on the opening edge of a corresponding one of the plate holding holes at three points. An engaging protrusion may be formed on one of one side face of each of the plastic plates and one side face of a corresponding one of the plate holding holes facing the one side face of each of the plastic plates, and an engaged recessed portion to be engaged with the engaging protrusion may be formed in the other of the one side face of each of the plastic plates and the one side face of the corresponding one of the plate holding holes. A heater housing portion may be formed in the reverse of each of the plastic plates so as to correspond to the recessed portions. Each of the recessed portions preferably has a sectional shape taken in depth directions thereof, the sectional shape being a trapezoid so that the opening portion of each of the recessed portions has a larger area than that of a bottom thereof. A large number of protrusions may be formed on a bottom of each of the recessed portions. Each of the plastic plates may have positioning means serving as a reference position when the samples are injected into the recessed portions. The samples may contain DNA fragments. Each of the plastic plates may be made of a resin containing a light absorbable material. A light absorbing coating may be formed on the surface of each of the plastic plates between adjacent two of the recessed portions. A light absorbable coating may be formed on the reverse of each of the plastic plates so as to separate the recessed portions from each other. Each of the recessed portions of each of the plastic plates preferably has a depth which is smaller than a width thereof. Moreover, a groove may be formed in the surface each of the plastic plates between adjacent two of the plurality of recessed portions. A groove surrounding each of the recessed portions may be formed in the surface of each of the plastic plates. The opening edge of each of the recessed portions may be formed so as to protrude from the surface of each of the plastic plates. A sample absorbing layer surrounding the opening edge of each of the recessed portions may be formed on the surface of each of the plastic plates.

### Brief Description of the Drawings

FIG. 1A is a plan view of the first preferred embodiment of a plastic plate according to the present invention, and FIG. 1B is an enlarged view of a portion A of FIG. 1A, FIG. 1C being a view of another embodiment of wells, and FIG. 1D being a view of a further embodiment of wells;
FIG. 2A is a sectional view taken along line IIA-IIA of wells of the first preferred embodiment of a plastic plate according to the present invention which is shown in FIGS. 1B and 1C, and FIG. 2B is a sectional view of another embodiment of wells;
FIG. 3A is abottomviewof a first modified example of the first preferred embodiment of a plastic plate according to the present invention, and FIG. 3B is an enlarged view of a portion B of FIG. 3A, FIG. 3C being a view of another embodiment of a heater housing portion;
FIG. 4A is a sectional view taken along line IVA-IVA of FIG. 3B, and FIG. 4B is a sectional view showing a state that a recess is formed in the reverse of the plastic plate of FIG. 2B for forming a heater housing portion;
FIG. 5A is a sectional view of wells of a second modified example of the first preferred embodiment of a plastic plate according to the present invention, and FIG. 5B is an enlarged view of a part (portion C) of FIG. 5A;
FIG. 6 is a plan view of the second preferred embodiment of a plastic plate assembly according to the present invention;
FIG. 7 is a plan view of a frame in the second preferred embodiment according to the present invention;
FIG. 8A is a plan view of the second preferred embodiment of a plastic plate according to the present invention, and FIG. 8B is a side view thereof, FIG. 8C being a bottom view thereof, FIG. 8D being an enlarged plan view of a part (portion D) of FIG. 8A, FIG. 8E being a sectional view taken along line VIIIE-VIIIE of wells of FIG. 8D, and FIG. 8F being a plan view showing another embodiment of wells;
FIG. 9 is a view showing the engagement of a plastic plate with a plate holding hole, which is viewed from the reverse side of the plastic plate;
FIG. 10A is a view showing the middle of the engagement of the plastic plate with the plate holding hole, which is viewed from the side, and FIG. 10B is a view showing a state that the engagement is completed;
FIG. 11A is a plan view showing a plate holding hole of a frame in the second preferred embodiment according to the present invention, and FIG. 11B is a sectional view taken along line XIB-XIB of FIG. 11A, FIG. 11C being a sectional view taken along line XIC-XIC of FIG. 11A;
FIG. 12A is a plan view of the third preferred embodiment of a plastic plate according to the present invention, and FIG. 12B is an enlarged view of a part (portion E) of the plastic plate of FIG. 12A;
FIG. 13 is a sectional view of wells of the plastic plate in the third preferred embodiment according to the present invention, which is taken along line XIII-XIII of FIG. 12B;
FIG. 14A is a bottom view of the fourth preferred embodiment of a plastic plate according to the present invention, and FIG. 14B is an enlarged view of a part (portion F) of the plastic plate of FIG. 14A;
FIG. 15 is a sectional view of wells of the plastic plate of FIG. 14B in the fourth preferred embodiment according to the present invention, which is taken along line XV-XV of FIG. 15B;
FIG. 16A is an enlarged view of a part of a plastic plate, which shows a first example of the sixthpreferred embodiment of a plastic plate according to the present invention, and FIG. 16B is a sectional view taken along line XVIB-XVIB of FIG. 16A;

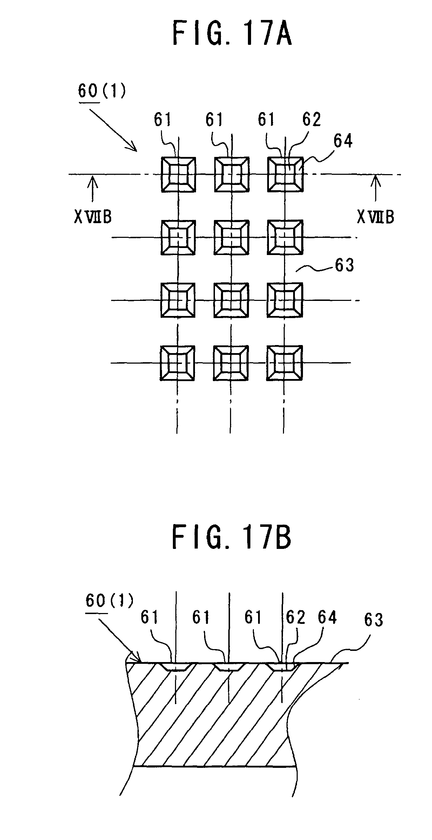

FIG. 17A is an enlarged view of a part of a plastic plate, which shows a second example of the sixth preferred embodiment of a plastic plate according to the present invention, and FIG. 17B is a sectional view taken along line XVIIB-XVIIB of FIG. 17A;
FIG. 18A is a plan view of the seventh preferred embodiment of a plastic plate according to the present invention, and FIG. 18B is an enlarged view of a portion G of FIG. 18A, FIG. 18C being a view of a first embodiment of wells, FIG. 18D being a view of a second embodiment of wells, and FIG. 18E being a third embodiment of wells;
FIG. 19A is a sectional view of a first embodiment of wells and grooves of the seventh preferred embodiment of a plastic plate according to the present invention, which is taken along line XIXA-XIXA of FIGS. 18B and 18D, and FIG. 19C is a sectional view of a second embodiment of wells and groove thereof, FIG. 19D being a sectional view of a third embodiment of wells and grooves thereof, and FIG. 19B being a sectional view of wells and grooves, which is taken along line XIXB-XIXB of FIG. 18E;
FIG. 20 is a sectional view of wells and grooves, which is taken along line XX-XX of FIG. 18C;
FIG. 21A is a plan view of a modified example of the seventh preferred embodiment of a plastic plate according to the present invention, and FIG. 21B is an enlarged plan view of a corner portion (portion H) of the plastic plate;
FIGS. 22A and 22B are sectional views of a groove 71 for showing other modified examples of the seventh preferred embodiment according to the present invention, FIG. 22A being a sectional view of a groove 71 having a substantially rectangular cross section, and FIG. 22B being a sectional view of a groove 71 having a substantially U-shaped cross section;
FIG. 23A is a plan view of the eighth preferred embodiment of a plastic plate according to the present invention, and FIG. 23B is an enlarged view of a portion I of FIG. 23A; and
FIG. 24 is a sectional view taken along line XXIV-XXIV of FIG. 23B.

### Best Mode for Carrying Out the Invention

Referring to the accompanying drawings, the preferred embodiments of a plastic plate and a plastic plate assembly according to the present invention will be described below in detail.

### [First Preferred Embodiment]

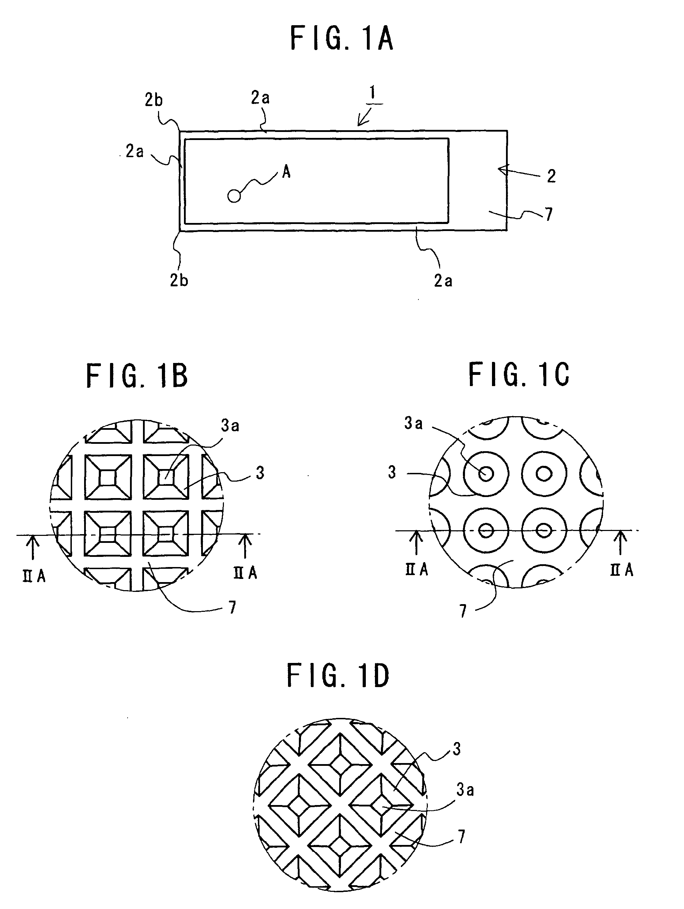

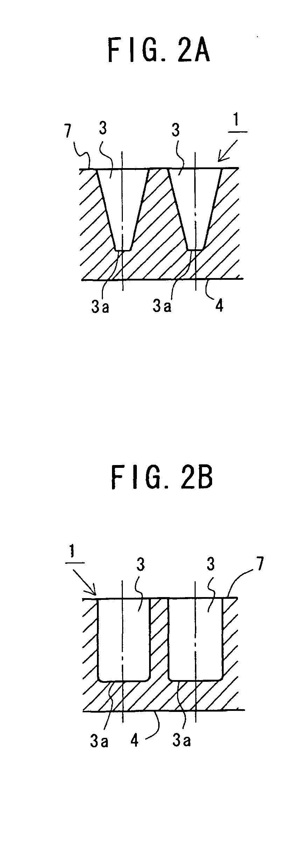

FIGS. 1A through 1D, 2A and 2B show a plastic plate 2 for a microarray 1 in this preferred embodiment according to the present invention. The sheet-like plastic plate 2 shown in these figures is made of, e .g., polycarbonate (PC), polymethyl methacrylate (PMMA) or an ultraviolet curable resin. The surface 7 of the plastic plate 2 has a large number of wells (recessed portions) 3 for housing therein a solution containing DNA fragments or specimens (e.g., cDNAs treated with a fluorescence dye). Each of the wells 3 is a recessed portion having a circular or square opening, the diameter or length of a side of which is in the range of from about 0.2 mm to about 1.0 mm, preferably from 0.2 mm to 0.5 mm. Thousands wells 3 are arranged at intervals of about 0.3 mm to about 1.0 mm, preferably 0.3 mm to 0.5 mm, to be formed in the rectangular flat surface substantially having the same size as that of a human thumb shown in FIG. 1A, so as to ensure a volume of tens nanoliters per well.

FIGS. 1B and 1C show examples of planar shapes of such wells 3. For example, each of the wells 3 shown in FIG. 1B has a quadrangular planar shape, and a substantially isosceles trapezoid cross section taken in depth directions (taken along line IIA-IIA of FIG. 1B), the cross section expanding upwards, and a flat bottom portion 3a (see FIG. 2A). That is, each of the wells 3 of FIG. 1B has a three-dimensional shape (a frustum of quadrangular pyramid) which is formed by cutting the top portion of a quadrangular pyramid in parallel to its bottom face and by inverting the cut quadrangular pyramid. Such wells 3 having a quadrangular planer shape (opening shape) can be more densely arranged since the dead space between the wells 3 is smaller than that between wells having a circular planer shape which will be described later. Each of the wells 3 shown in FIG. 1C has a circular planer shape, and a substantially isosceles trapezoid cross section taken in depth directions (taken along line IIA-IIA of FIG. 1C), the cross section expanding upwards, and a flat bottom face 3a (see FIG. 2A). That is, each of the wells 3 shown in FIG. 1C has a three-dimensional shape (a frustum of circular cone) which is formed by cutting the top portion of a circular cone in parallel to its bottom face and by inverting the cut circular cone. Furthermore, the shape of each of the wells 3 should not be limited to that in the above described embodiments, but it may be another shape. For example, each of the wells 3 may have a substantially rectangular cross section as shown in FIG. 2B. Alternatively, as shown in FIG. 1D, the wells 3 having a square planar shape may be arranged so that the diagonal lines of the wells 3 extend in parallel to the sides of the plastic plate 2. In other words, the wells 3 may be arranged so that the wells 3 shown in FIG. 1B are rotated by 45° . If the wells 3 are thus arranged, the length of the opening of each of the wells 3 in lateral and longitudinal directions of FIG. 1A can be longer than that of FIG. 1B. Moreover, the planer shape (opening shape) of each of the wells 3 may be polygonal, such as triangular, rectangular or hexagonal, or circular, such as elliptic or flat oval.

The plastic plate 2 having such wells 3 is formed by, e.g., the injection molding method for injecting a molten resin into a cavity of a metal pattern to transfer the shape of the metal pattern, or the compression molding method for filling resin powder in a metal pattern to heat and press the resin powder to transfer the shape of the metal pattern, or a molding method for applying an ultraviolet curable resin on the surface of a metal pattern to irradiate the resin with ultraviolet rays to cure the resin to transfer the shape of the metal pattern.

Then, a very small amount of DNA is housed in each of the wells 3 of the plastic plate 2 thus formed, and a specific site of the DNA is amplified in a short time by the PCR method (the polymerase chain reaction method) to form the microarray 1 wherein thousands wells 3 housing therein a sufficient amount of DNA fragment (gene fragment) are densely arranged. Thereafter, a specimen (cDNA) treated with the fluorescence dye is injected into each of the wells 3 of the microarray 1 to be hybridized with the DNA fragment amplified in each of the wells 3 of the plastic plate 2. Then, the cDNA in each of the wells 3 of the microarray 1 is irradiated with light beams exciting the fluorescence dye, and fluorescence is detected by a photo detector. Thus, it is possible to identify the specimen hybridized with the DNA fragment, so that it is possible to reveal the function of each of genes.

The plastic plate 2 for the microarray 1 in this preferred embodiment as described above can house a sufficient amount of DNA fragment, specimen and reagent in each of the wells 3, so that it is possible to more precisely analyze genes.

Since the plastic plate 2 in this preferred embodiment can amplify the DNA in each of the wells 3, the plastic plate 2 can also be used as a reactor in the PCR method. Thus, it is not required to provide any reactor in the PCR method which is required in the conventional microarray technique, and it is not required to apply DNA fragments on a glass plate from the reactor, so that it is possible to reduce the costs of equipment and operation time.

The plastic plate 2 in this preferred embodiment can be small since the distance between adjacent two of the wells 3 is small. In addition, since the plastic plate 2 has a low heat capacity, the response to heating or cooling is excellent, so that it is possible to greatly contribute to the shortening of time in the PCR method and contribute to the reduction of the size of the system.

In order to thus improve the response to heat, the plastic plate 2 may be formed of a resin material having a high heat conductivity.

It could be considered to irradiate a glass plate with laser beams to partially melt the glass plate to form wells. However, it takes a lot of time to prepare the wells in comparison with this preferred embodiment wherein the shape of the metal pattern is transferred by the injection molding method or the like. In addition, the price of the plate is more expensive than that of the plastic plate 2 in this preferred embodiment, and the variation in shape of the wells is greater than that of the plastic plate 2 in this preferred embodiment. That is, according to this preferred embodiment, it is possible to provide the inexpensive plastic plate 2 for the microarray 1 with the wells 3 having a precise shape (dimension).

If each of the wells 3 of the plastic plate 2 in this preferred embodiment has a substantially isosceles trapezoid cross section as shown in FIG. 2A, the bottom portion 3a can be flat so as to be suited for heat transfer, and the mold releasing capability from the metal pattern can be improved. Therefore, when the DNA in each of the wells 3 is amplified by the PCR method, the heat of a heater (not shown) arranged on the side of the reverse 4 of the plastic plate 2 is easily transferred into a corresponding one of the wells 3, and the plastic plate 2 including the wells 3 can be precisely molded.

Furthermore, as shown in FIG. 1A, the frame-like portion 2a formed around the wells 3 so as to be integrated with the wells 3 may have a width of at least 0.5 mm or more, preferably 2.0 mm or more, and a thickness of at least 0.5 mm, preferably 1.0 mm, in order to ensure the precision of position (rigidity) of the wells 3 and in view of internal stress acting on the corner portions 2b.

### (First Modified Example of First Preferred Embodiment)

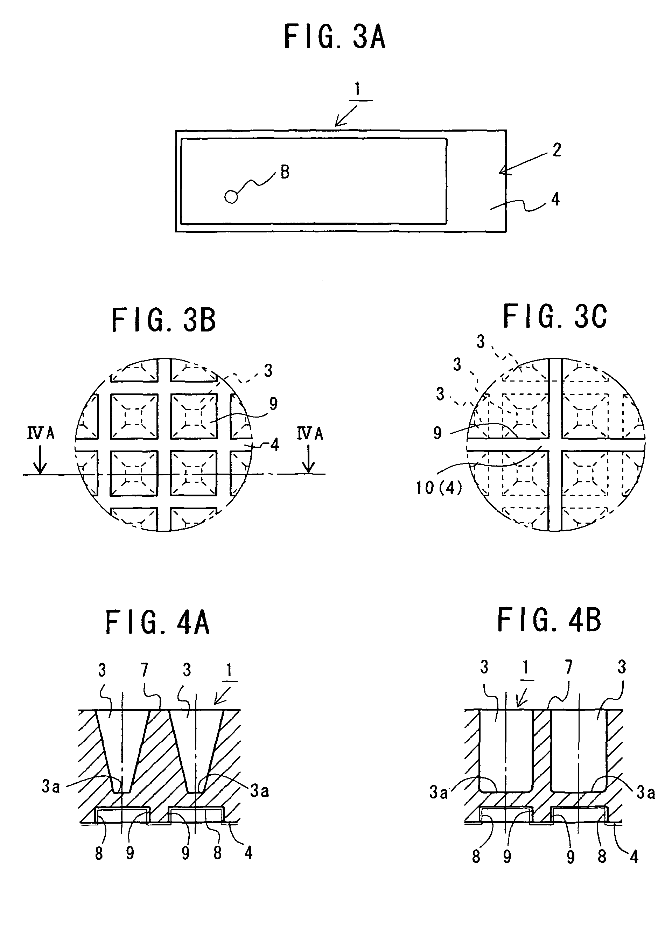

FIGS. 3A through 3C, 4A and 4B show a first modified example of this preferred embodiment. As shown in these figures, a portion of the reverse 4 of the plastic plate 2 facing each of the wells 3 is recessed so as to form a heater housing portion 9 having a predetermined size for housing therein a heater 8, which is arranged on the side of the reverse 4 of the plastic plate 2, while leaving a rib 10 around the heater housing portion 9. With such a construction, if the DNA or the like housed in each of the wells 3 is heated by the PCR method, the heat of the heater 8 is easily transferred to the DNA or the like in each of the wells 3 since the reverse 4 of the plastic plate 2 is recessed to form the heater housing portions 9 to decrease the thickness of the plastic plate 2 between the bottom portion 3a of each of the wells 3 and a corresponding one of the heaters 8. Therefore, according to this modified example, the amplification of the DNA in each of the wells 3 can be promoted, so that the time to amplify the DNA can be shortened.

The number of the heater housing portions 9 shown in FIG. 3B is equal to the number of the wells 3. In other words, the ribs 10 are formed on the reverse 4 so as to surround each of the wells 3. The heater housing portions 9 shown in FIG. 3C are formed so as to correspond to a plurality of wells 3. In other words, the ribs 10 are formed so as to surround the plurality of wells 3. If the ribs 10 are thus formed on the reverse 4 of the plastic plate 2 so as to surround the wells 3, the thickness of the plastic plate 2 between the wells 3 and the reverse 4 can be small to such an extent (to, e.g., about 0.2 mm to about 0.4 mm in the case of the shape shown in FIG. 3B) that the plastic plate 2 can be molded while the rigidity thereof is ensured.

### (Second Modified Example of First Preferred Embodiment)

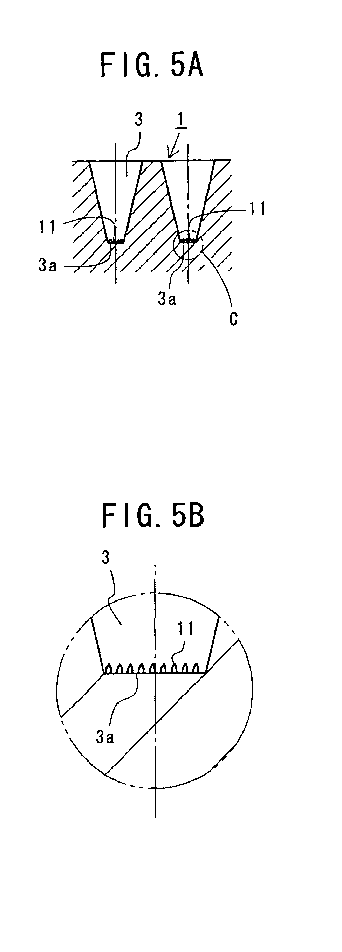

FIGS. 5A and 5B show a second modified example of this preferred embodiment. In this example, a plurality of protrusions 11 are formed on the bottom portion 3a of each of the wells 3 to increase the heat transfer area of the bottom portion 3a. Thus, the heat of a heater (not shown) is easily transferred to a DNA or the like in each of the wells 3, and the convection of a solution in each of the wells 3 is promoted, so that the amplification of the DNA in each of the wells 3 is further promoted. Therefore, it is possible to shorten the time to amplify the DNA.

The plurality of protrusions annularly arranged may be arranged concentrically with each other although such arrangement is not shown.

One or a plurality of protrusions may be columnar so as to extend to the middle or top portion of each of the wells 3.

### [Second Preferred Embodiment]

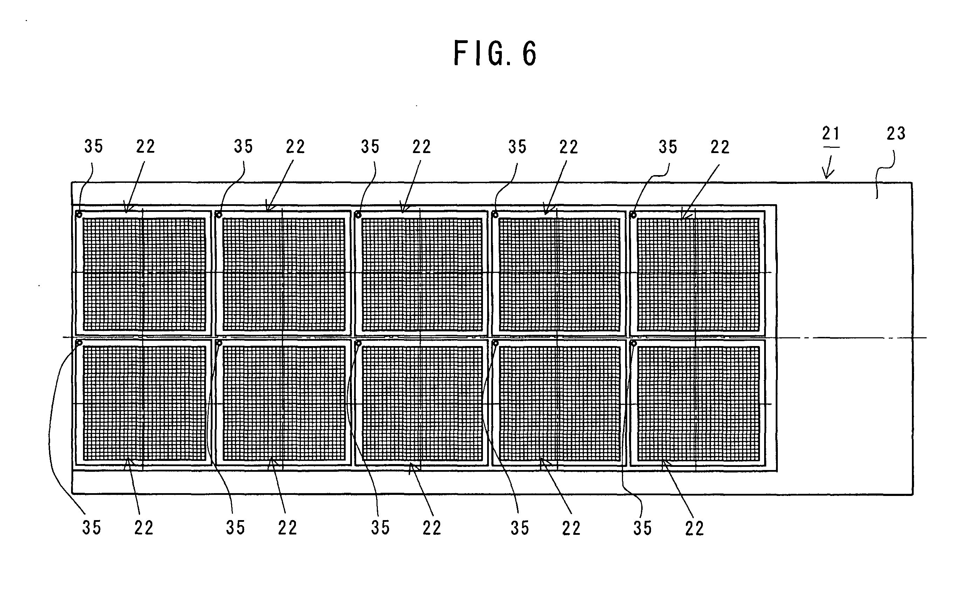

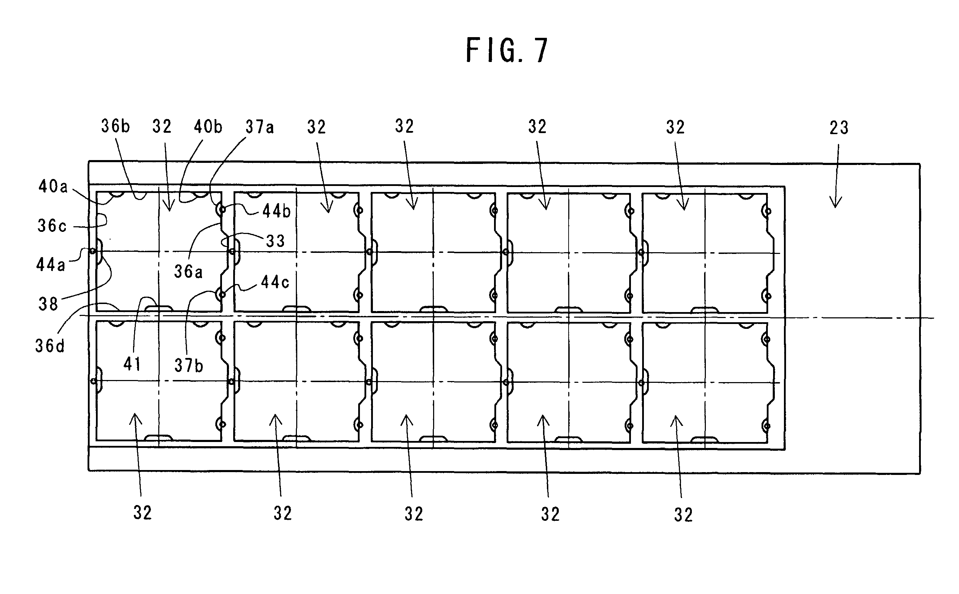

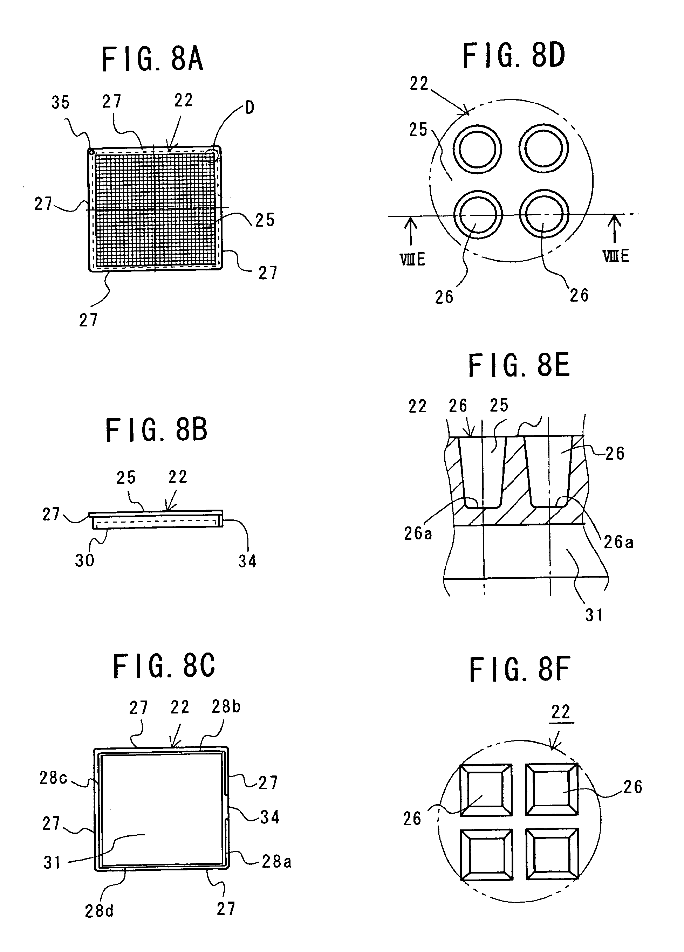

FIGS. 6, 7 and 8A through 8F show a plastic plate assembly 21 for a microarray in the second preferred embodiment of the present invention. FIG. 6 is a plan view of the plastic plate assembly 21, and FIG. 7 is a plan view of a frame 23 of the plastic plate assembly 21 from which plastic plates 22 are removed. FIGS. 8A through 8F are views showing one of the plastic plates 22.

As shown in these figures, the plastic plate assembly 21 in this preferred embodiment comprises ten plastic plates 22 having a substantially rectangular planar shape, and a frame 23 for holding the plastic plates 22 at predetermined positions.

The plastic plate assembly 21 is designed to divide the plastic plate 2 in the above described first preferred embodiment into a plurality of parts. The plastic plates 22 are formed of the same resin material as that of the plastic plate 2 in the above described first preferred embodiment, substantially by the same forming method as that in the first preferred embodiment.

That is, as shown in FIGS. 8A through 8F in detail, the plastic plate 22 has a large number of wells (recessed portions) 26 in the surface (top face) 25 thereof. The plastic plate 22 has a flange portion 27 which extends over the entire periphery thereof on the side of the surface 25 and which protrudes from the side faces 28a through 28d thereof. On the side of the reverse 30 of the plastic plate 22, a rectangular heater housing portion 31 is formed so as to be recessed, so that the thickness of the plastic plate 22 between the bottom portions 26 of all of the wells 26 and the heater housing portions 31 is small so as to be suited for heat transfer.

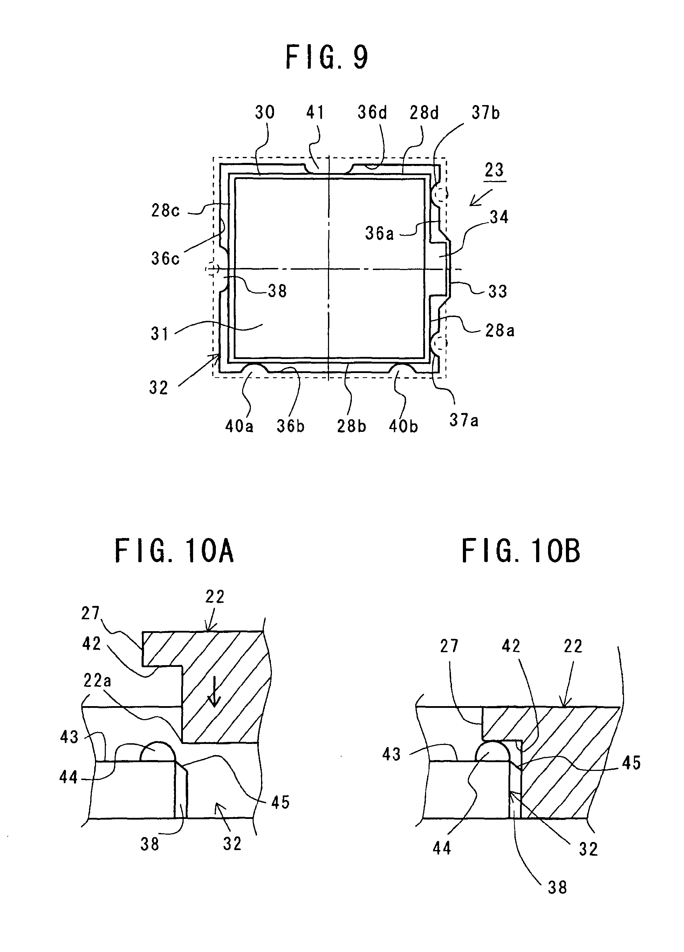

On one side face 28a of the plastic plate 22, there is formed an engaging protrusion 34 for engaging an engaged recessed portion 33 of a plate holding hole 32 of the frame 23 to prevent erroneous assembly. That is, when the plastic plate 22 is mounted on the frame 23, if the plastic plate 22 is inserted into the plate holding hole 32 of the frame 23 so that the engaging protrusion 34 engages the engaged recessed portion 33 of the frame 23, it is possible to prevent the plastic plate 22 from being mounted on the frame 23 in an erroneous direction, so that the plastic plate 22 can be surely mounted in the plate holding hole 32 of the frame 23 (see FIGS. 8A through 8F and 9) . Furthermore, as shown in FIGS. 8D through 8F, each of the wells 26 substantially has the same shape as that of each of the wells 3 in the above described first preferred embodiment, i.e., the shape of a frustum of circular cone or quadrangular pyramid. FIG. 8D corresponds to FIG. 1C, and FIG. 8E corresponds to FIG. 2A, FIG. 8F corresponding to FIG. 1B.

The frame 23 is formed of a plastic, glass or metal (e.g., aluminum or stainless). The same number of the substantially rectangular plate holding holes 32 as the number of the plastic plates 22 are formed in the frame 23 for holding the plastic plate 22 as described above.

As shown in FIGS. 6 and 8A, the plastic plate 22 has a positioning hole 35 serving as a positioning means in a corner portion of the top face thereof. The positioning hole 35 serves as a reference position on a coordinate plane for operating a spotting device, which is used for injecting a DNA fragment and/or specimen into each of the wells 26, and for operating a photo detector which will be described later. If the positioning hole 35 is detected by a sensor when the spotting device or the photo detector is operated, the operation reference position can be determined every plastic plate 22, and errors caused by mounting the plastic plates 22 in the frame 23 can be modified, so that each operation, such as spotting or photo detecting operation, can be precisely carried out. Furthermore, the positioning hole 35 is an example of a positioning means, and the present invention should not be limited thereto, but a light reflecting substance or magnetic substance capable of being detected by a photo sensor or magnetic sensor may be provided. While one positioning hole 35 has been formed in the upper-left corner portion in FIG. 8A in this preferred embodiment, the present invention should not be limited thereto. For example, a plurality of positioning holes may be formed in the corner portions on a diagonal line (the lower-right corner portion in addition to the upper-left corner portion) to more precisely position the coordinate plane every plastic plate 22.

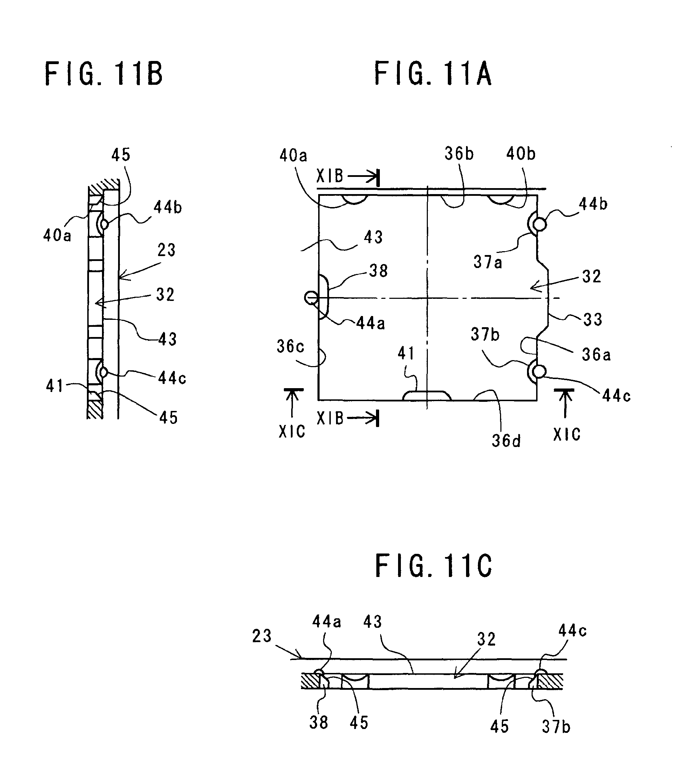

As shown in FIGS. 7 and 11A, the side faces 36a and 36c of the plate holding hole 32 facing each other have protrusions 37a, 37b and 38 for supporting thereon the facing side faces 28a and 28c of the plastic plate 22 at three points. That is, the plate holding hole 32 shown in FIGS. 7 and 11A has the protrusion 38 on the left side face 36c at the substantially central portion thereof, and the protrusions 37a and 37b on both end portions of the right side face 36a. The three protrusions 38, 37a and 37b are arranged so as to form an isosceles triangle, the vertex of which corresponds to the protrusion 38, to hold the facing side faces 28a and 28c of the plastic plate 22 at three points (see FIG. 9). Furthermore, FIG. 9 shows the reverse 30 of the plastic plate 22 mounted in the plate holding hole 32.

As shown in FIGS. 7 and 11A, the other side faces 36b and 36d of the plate holding hole 32 facing each other have protrusions 40a, 40b and 41 for supporting thereon the other facing side faces 28b and 28d of the plastic plate 22 at three points. That is, the plate holding hole 32 shown in FIGS. 7 and 11A has the protrusion 41 on the lower face 36d at the substantially central portion thereof, and the protrusions 40a and 40b on both end portions of the upper face 37b. The three protrusions 41, 40a and 40b are arranged so as to form an isosceles triangle, the vertex of which corresponds to the protrusion 41, to hold the other facing side faces 28b and 28d of the plastic plate 22 at three points (see FIG. 9).

As shown in FIGS. 7, 10B and 11A through 11C, a surface 43 of the plastic plate 22 to face the bottom face 42 of the flange portion 27 has protrusions 44a, 44b and 44c for supporting thereon the flange portion 27 at three points. That is, the frame 23 has one protrusion 44a in the vicinity of the protrusion 38, and a pair of protrusions 44b and 44c in the vicinity of the protrusions 37a and 37b, respectively, so that the three protrusions 44a, 44b and 44c are arranged so as to form an isosceles triangle, the vertex of which corresponds to the protrusion 44a.

Thus, according to this preferred embodiment, the side faces 28a through 28d and flange portion 27 of the plastic plate 22 are designed to be supported on the protrusions 37a, 37b and 38, 40a, 40b and 41, 44a, 44b and 44c of the frame 23, at three points, respectively, so that the plastic plate 22 can be precisely held in the frame 23 without being influenced by the profile irregularity (waviness or the like) of the side faces 36a through 36d of the plate holding hole 32 of the frame 23 and by the profile irregularity (waviness or the like) of the face 43 of the opening edge portion of the plate holding hole 32 of the frame 23.

As shown in FIGS. 7 and 10A, the C-chamfering of the top portion of each of the protrusions 37a, 37b, 38, 40a, 40b and 41 formed on the side faces 36a through 36d of the plate holding hole 32 is carried out to form inclined surfaces 45 on the top portion of each of the protrusions 37a, 37b, 38, 40a, 40b and 41. Thus, as shown in FIG. 10A, even if the plastic plate 22 is slightly displaced with respect to the plate holding hole 32 to be inserted into the plate holding hole 32, the corner portions 22a of the plastic plate 22 are guided on the inclined surfaces 45, so that the plastic plate 22 can be easily mounted in the plate holding hole 32.

Then, in this preferred embodiment, after ten plastic plates 22 having a large number of wells 26 are mounted in the plate holding holes 32 of the frame 23, respectively, to assemble the plastic plate assembly 21, a very small amount of DNA is housed in each of the wells 26 of each of the plastic plates 22, and a specific site of the DNA is amplified in a short time by the PCR method (polymerase chain reaction method) to form a microarray. Thereafter, a specimen (cDNA) treated with a fluorescence dye is injected into each of the wells 26 of the microarray, so that the specimen and DNA fragment are hybridized in each of the wells 26. Then, the cDNA in each of the wells 26 of the mircoarray is irradiated with light beams exciting the fluorescence dye, and fluorescence is detected by a photo detector. Thus, it can be determined which specimen has been hybridized with the DNA fragment, so that the function of each gene can be verified.

Similar to the plastic plate 2 in the above described first preferred embodiment, the plastic plate assembly 21 for microarray in this preferred embodiment described above can house a solution containing a sufficient amount of DNA fragment, specimen and reagent in each of the wells 3, so that it is possible to more precisely analyze genes.

Similar to the plastic plate 2 in the above described first preferred embodiment, the plastic plate assembly 21 in this preferred embodiment can amplify the DNA in each of the wells 26, so that the plastic plate assembly 21 can also be used as a reactor in the PCR method. Thus, it is not required to provide any reactor in the PCR method which is required in the conventional microarray technique, and it is not required to apply DNA fragments on a glass plate from the reactor, so that it is possible to reduce the costs of equipment and operation time.

Since the plastic plate assembly 21 in this preferred embodiment is formed by mounting the plurality of plastic plates 22 in the frame 23, a metal pattern for forming each of the plastic plates 22 is smaller than a metal pattern for forming the plastic plate 2 in the above described first preferred embodiment. Therefore, the metal pattern itself can be easily produced, so that it is possible to reduce the costs of producing the metal pattern. As a result, according to this preferred embodiment, the price of the plastic plate assembly 21 can be lower than that of the above described plastic plate 2.

The reverse 30 of each of the plastic plates 22 in this preferred embodiment has a recessed heater housing portion 31 to easily transfer the heat of a heater (not shown) into each of the wells 26, so that it is possible to rapidly and efficiently amplify DNAs by the PCR method.

Furthermore, the protrusions 11 shown in FIGS. 5A and 5B may be applied to each of the wells 26 of the plastic plates 22 in this preferred embodiment. While all of the protrusions 37a, 37b, 38, 40a, 40b, 41, 44a, 44b and 44c for supporting thereon each of the plastic plates 22 at three points have been formed on the frame 23 in this preferred embodiment, at least one of the plastic plate 22 and the frame 23 may have these protrusions. While each of the plastic plates 22 has been supported on the three protrusions of the frame 23 at three points in this preferred embodiment, the present invention should not be limited thereto. For example, each of the plastic plates 22 may be supported at points of more than three, such as four points. Moreover, while the ten plastic plates 22 have been held in the frame 23 on the plastic plate assembly 21 in this preferred embodiment, the present invention should not be limited thereto. The size and number of the plastic plates 22 may be suitably changed in accordance with an analyzing device to be used.

### [Third Preferred Embodiment]

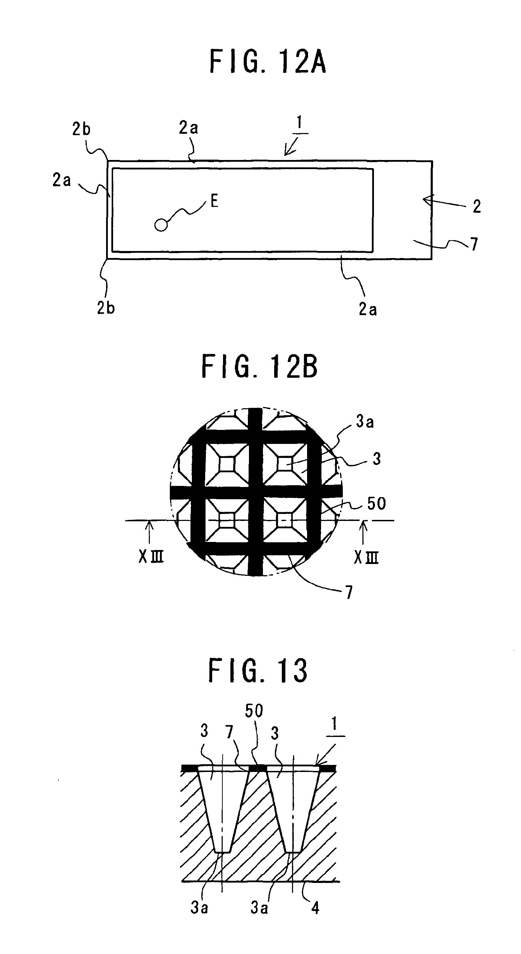

FIGS. 12A, 12B and 13 show the third preferred embodiment of a plastic plate 2 according to the present invention as an applied example of the plastic plate 2 in the first preferred embodiment. Furthermore, in this preferred embodiment, the same reference numbers as those in the plastic plate 2 in the above described first preferred embodiment are given to the same portions as those in the first preferred embodiment to omit repeated explanation.

In the plastic plate 2 shown in these figures, a paint containing a light absorptive material is applied on the surface 7 between the wells 3 by a well-known printing technique, such as the silk (screen) printing, to form a substantially lattice-shaped light absorptive coating 50 surrounding each of the wells 3. Although the color of the paint should not be limited as long as the paint is light-absorptive, it is preferably black in order to prevent a background value from rising during scanning and in order to improve the precision of measurement.

According to this preferred embodiment with such a construction, even if the hybridized cDNA in each of the wells 3 of the plastic plate 2 for the microarray 1 is irradiated with fluorescence dye exciting light beams from the reverse 4 of the plastic plate 2 to allow the cDNA in each of the wells 3 to fluoresce, the lattice-shaped light absorptive coating 50 formed on the surface 7 of the plastic plate 2 can inhibit the fluorescent light from traveling toward the surface 7 above the next well 3. That is, according to this preferred embodiment, the light absorptive coating 50 can prevent the crosstalk of light between adjacent wells 3, so that it is possible to precisely detect fluorescent emission by a photo detector (not shown) arranged on the side of the surface 7 of the plastic plate 2.

While the opening portion of each of the wells 3 has been rectangular as an example in this preferred embodiment, the opening portion of each of the wells 3 of the plastic plate may have a circular shape shown in FIG. 1C or another shape, and the light absorptive coating 50 may be formed on the surface 7 between adjacent two of such wells 3, each of which has an opening portion having a shape other than rectangles. Moreover, this preferred embodiment may be applied to the plastic plate 22 in the second preferred embodiment shown in FIGS. 8A through 8F.

### [Fourth Preferred Embodiment]

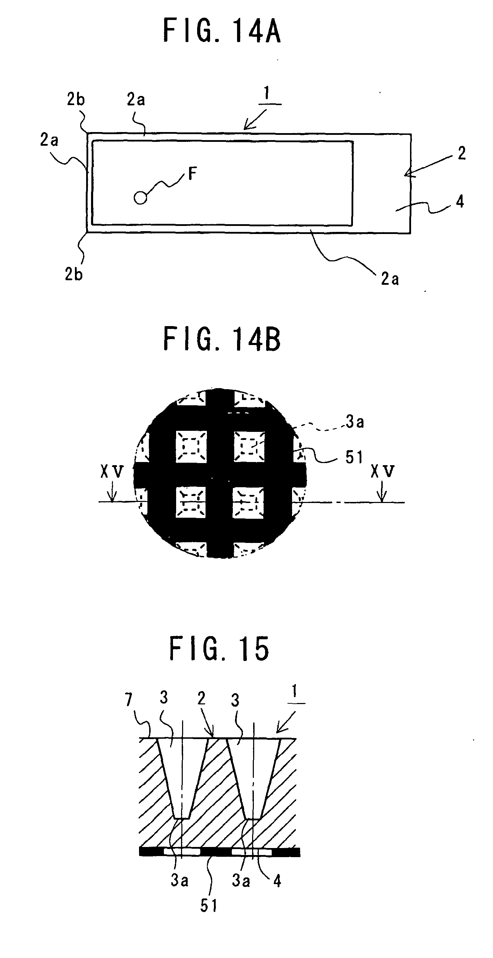

FIGS. 14A, 14B and 15 show the fourth preferred embodiment of a plastic plate 2 according to the present invention as an applied example of the plastic plate 2 in the first preferred embodiment. Furthermore, in this preferred embodiment, the same reference numbers as those in the plastic plate 2 in the above described first preferred embodiment are given to the same portions as those in the first preferred embodiment to omit repeated explanation.

In this plastic plate 2, a paint containing a light absorptive material is applied on the reverse 4 so as to surround the bottom portion 3a of each of the wells 3 by the well-known silk (screen) printing or the like to form a lattice-shaped light absorptive coating 51 on the reverse 4. Similar to the above described third preferred embodiment, although the color of the paint should not be limited as long as the paint is light-absorptive, it is preferably black in order to prevent a background value from rising during scanning and in order to improve the precision of measurement.

In this preferred embodiment, light transmitting windows formed by the light absorptive coating 51 are formed so as to have such a size that it is possible to prevent crosstalk until light beams, with which the reverse 4 of the plastic plate 2 is irradiated, reach the photo detector arranged on the side of the surface 7 and that it is possible to transmit a sufficient quantity of light to allow detection by the photo detector arranged on the side of the surface 7. For example, it is considered that each of the windows is formed so as to be larger than at least the bottom portion 3a of a corresponding one of the wells 3 and so as to be smaller than the opening portion of the corresponding one of the wells 3.

According to this preferred embodiment with such a construction, if the hybridized cDNA in each of the wells 3 of the plastic plate 2 for the microarray 1 is irradiated with light beams exciting fluorescence dye, the light absorptive coating 51 formed on the reverse 4 can be previously adjusted so as to allow light beams to enter only the wells 3. That is, according to this preferred embodiment, the light absorptive coating 51 can prevent the crosstalk of transmitted light between adjacent wells 3, so that it is possible to precisely detect fluorescent emission by the photo detector (not shown) arranged on the side of the surface 7 of the plastic plate 2.

While the opening portion of each of the wells 3 has been rectangular as an example in this preferred embodiment, the opening portion of each of the wells 3 of the plastic plate may have a circular shape shown in FIG. 1C or another shape, and the light absorptive coating 51 may be formed on the reverse 4 between adjacent two of such wells 3, each of which has an opening portion having a shape other than rectangles. Moreover, this preferred embodiment may be applied to the plastic plate 22 in the second preferred embodiment shown in FIGS. 8A through 8F.

### [Fifth Preferred Embodiment]

The plastic plates 2 and 22 in the above described first and second preferred embodiments may be formed by injection molding using a black plastic containing a light absorptive material (preferably, e.g., carbon black).

Thus, even if the hybridized cDNA in each of the wells 3, 26 of the plastic plate 2, 22 is irradiated with fluorescence-dye exciting light beams to allow the cDNA in each of the wells 3, 26 to fluoresce, the light absorptive material contained in the plastic plate 2, 22 can inhibit the fluorescent light from traveling toward the surface 7, 25 above the next well 3, 26 and toward the reverse 4, 30 below the next well 3, 26. That is, according to this preferred embodiment, it is possible to prevent the crosstalk of light between adjacent wells 3, 26, so that it is possible to precisely detect fluorescent emission by a photo detector (not shown) arranged on the side of the surface 7, 25 of the plastic plate 2, 22.

### [Sixth Preferred Embodiment]

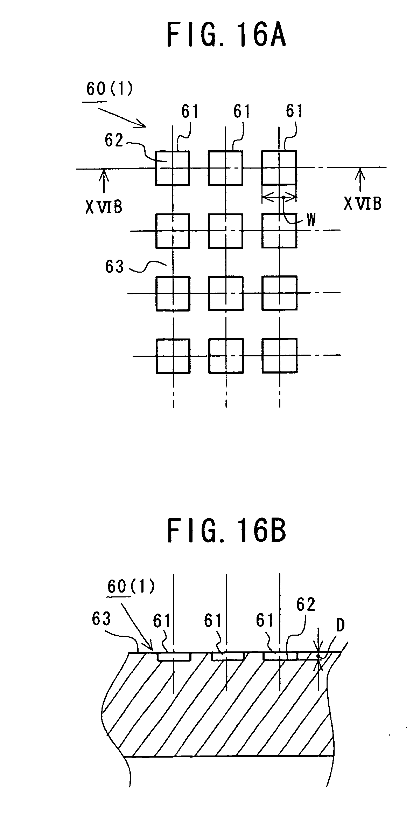

FIGS. 16A and 16B show the sixth preferred embodiment of a plastic plate 60 for a microarray 1 according to the present invention. As shown in FIGS. 16A and 16B, the cross sectional shape of each of wells 61 of the plastic plate 60 in this preferred embodiment is different from that in the above described preferred embodiments. That is, in the plastic plate 60 in this preferred embodiment, the depth (D) of each of the wells 61 is smaller than the length (W) of one side of the opening portion of each of the wells 61 having a substantially square planar shape (e.g., W/D ≒ 5 in this preferred embodiment). Each of the wells 61 of the plastic plate 60 is formed so as to have a content volume of about hundreds picoliters.

In the plastic plate 60 in this preferred embodiment, a cultured cell bonding material (e.g., a glycoprotein, such as collagen, gelatin, fibronectin or laminin, or a synthetic peptide, such as poly-L-lysine) may be previously applied on the bottom face 62 of each of the wells 61 to enhance the adhesive property of the cultured cell or the like injected from a nozzle of an ink jet head. Alternatively, the plastic plate 60 may be charged to be plus while DNA fragments or the like may be changed to be minus, so that the DNA fragments or the like may be electrically absorbed and fixed in the wells 61 of the plastic plate 60.

With such a construction, if a protective coating is formed on the surface 63 of the plastic plate 60 in order to prevent dust or the like in the atmosphere from adhering to DNA fragments or the like in the wells 61, even if the protective coating is washed out during measurement, it is possible to absorb and fix the DNA fragments or the like in the wells 61, so that it is possible to prevent the DNA fragments or the like in the wells 61 from escaping.

In the plastic plate 60 for the microarray 1 in this preferred embodiment with such a construction, when DNA fragments or the like are spotted onto a plastic or glass plate by utilizing the ink jet technique for printers, DNA fragments or the like (the volume of a drop is, e.g., about 20 to 30 picoliters) injected from the nozzle of an ink jet head can be surely acquired into the wells 61 to prevent the DNA fragments or the like to be arranged in adjacent wells 61 from being mixed with each other, so that it is possible to precisely carry out measurements.

Since the depth D of each of the wells 61 of the plastic plate 60 in this preferred embodiment is smaller than that of each of the plastic plates 2 and 22 in the above described preferred embodiments, the interior of the wells 61 can be easily washed. The plastic plate 60 can be washed by a well-known means or technique using service water or ultrapure water.

While each of the wells 61 of the plastic plate 60 in this preferred embodiment has a substantially rectangular cross section, it may have a substantially trapezoid cross section so that the facing side faces 64 gradually expand from the bottom face 62 to the opening portion thereof. Thus, the plastic plate 60 can be easily released from an injection molding die.

Furthermore, the term "width W" of each of the wells 61 means the length of one side of each of the wells 61 when the planar shape of the opening portion thereof is square as described above, or the length of a long side of each of the wells 61 when the planar shape of the opening portion thereof is rectangular, or the diameter of each of the wells 61 when the planar shape of the opening portion thereof is circular, or the length of the longest side or longest diameter of each of the wells 61 when the opening portion thereof has another planar shape.

### [Seventh Preferred Embodiment]

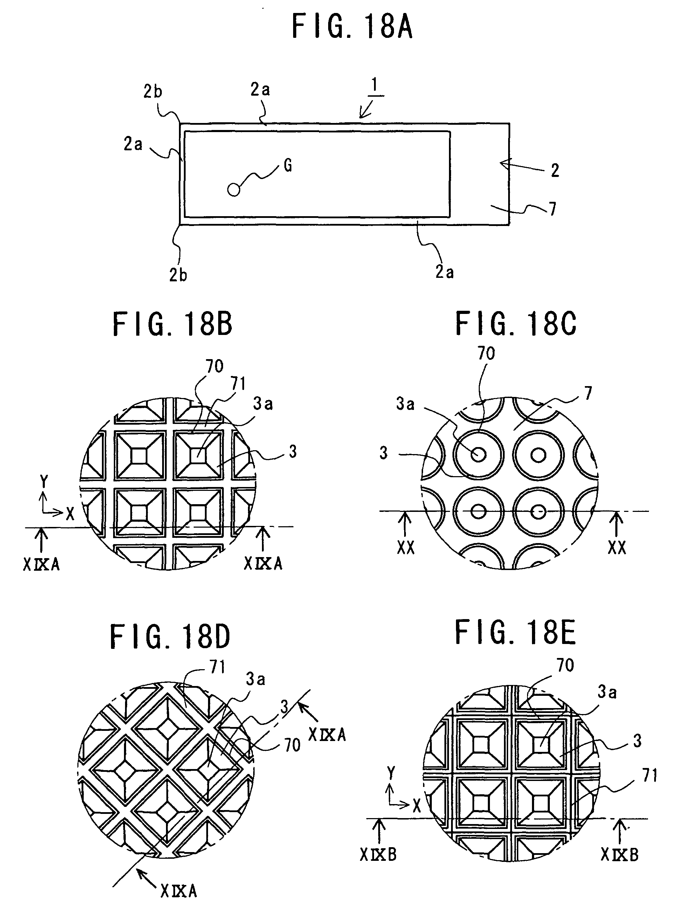

FIGS. 18A through 18E, 19A through 19D and 20 show the seventh preferred embodiment of a plastic plate 2 for a microarray 1 according to the present invention as an applied example of the plastic plate 2 in the first preferred embodiment. Furthermore, in this preferred embodiment, the same reference numbers as those in the plastic plate 2 in the above described first preferred embodiment are given to the same portions as those in the first preferred embodiment to omit repeated explanation.

As shown in FIGS. 18A, 18B, 18D and 18E, in the surface 7 of a plastic plate 2 having a large number of wells 3, each of which has an opening portion having a rectangular planer shape, a groove 71 is formed between adjacent two of the wells 3.

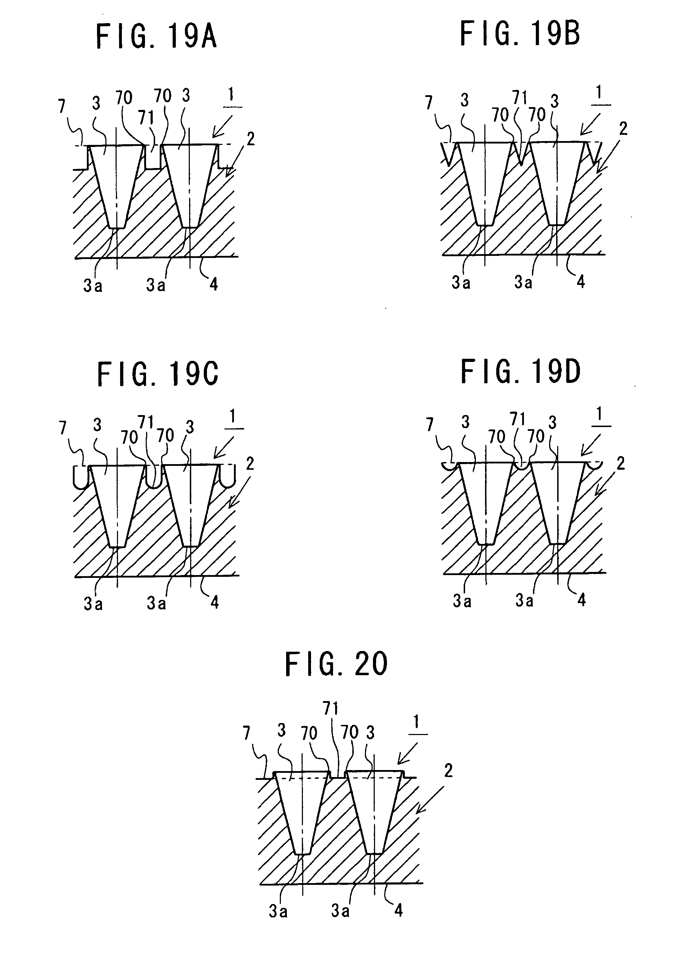

The sectional shape of each of the grooves 71 formed in the plastic plate 2 should not be limited to a specific shape. For example, the sectional shape thereof may be a rectangle as shown in FIG. 19A, or a substantially U-shape as shown in FIG. 19C, or a semi-circle as shown in FIG. 19D. Alternatively, as shown in FIGS. 18E and 19B, in the plastic plate 2 having a large number of wells 3 having an opening portion having a quadrangular planar shape, the sectional shape of the groove 71 formed between adjacent two of the wells 3 may be a triangle. The optimum width and depth of the groove 71 are set in accordance with the amount of a sample to be injected into the wells 3, the distance between adjacent two of the wells 3, and so forth.

As shown in FIG. 18C, in a plastic plate 2 having a large number of wells 3, each of which has an opening portion having a circular planar shape, the opening edge 70 of each of the wells 3 protrudes from the surface 7 of the plastic plate 2 by a predetermined height (see FIG. 20). The optimum height of the opening edge 70 of each of the wells 3 protruding from the surface 7 of the plastic plate 2 is set in accordance with the amount of a sample to be injected into the wells 3, the distance between adjacent two of the wells 3, and so forth. Furthermore, the opening edge 70 of each of the wells 3 shown in FIGS. 18B, 18D and 18E may protrude from the surface 7 as shown in FIG. 20.

According to this preferred embodiment with such constructions, when a sample is injected into each of the wells 3, even if the position of the injected sample is displaced so that the sample drops into a space between adjacent two of the wells 3, the sample can be housed in the groove 71 between the adjacent two of the wells 3 (see FIGS. 19A through 19D), or the sample can be held on the surface 7 between the adjacent two of the wells 3 (see FIG. 20). As a result, when different kinds of samples are injected into adjacent two of the wells 3, respectively, the samples incorrectly injected into the wells 3 are housed in the groove 71 between the adjacent two of the wells 3 or held on the surface 7 between the adjacent two of the wells 3. Thus, it is possible to prevent the samples from being erroneously injected into wells 3 other than wells 3 into which the samples are to be originally injected, and it is possible to prevent the different kinds of samples from being mixed with each other. Moreover, if the plastic plate 2 in this preferred embodiment is used for carrying out quantitative analysis or the like, the samples incorrectly injected into the wells 3 can be housed in the grooves 71 between the wells 3 or held on the surface 7 between the wells 3. Thus, even if a predetermined amount of sample has been incorrectly injected into one of adjacent two of the wells 3, an excessive amount of sample incorrectly injected is not excessively inj ected into the other well of the adj acent two of the wells 3. That is, it is possible to ensure a predetermined amount of sample to be injected into the other well 3 than the well 3 into which the predetermined amount of sample has been incorrectly injected.

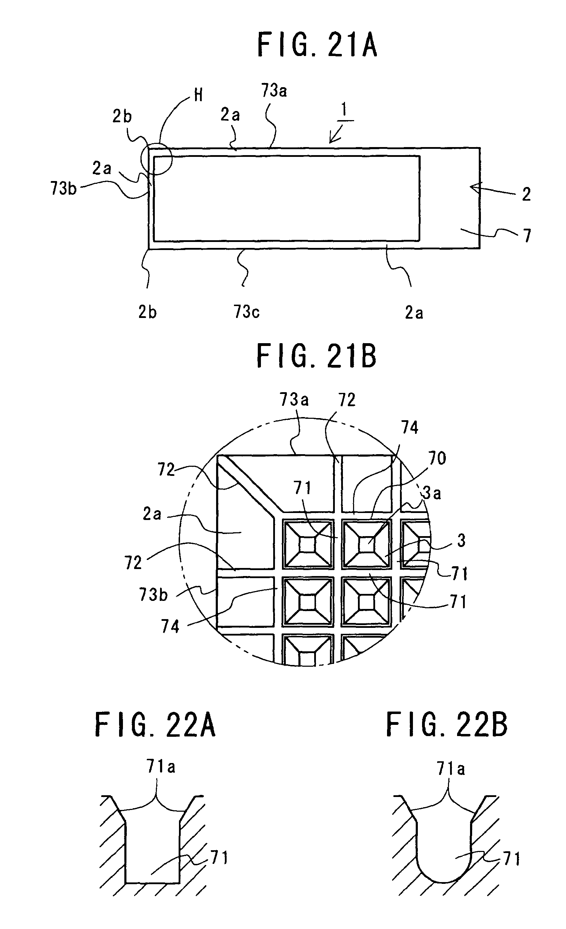

As shown in FIGS. 21A and 21B, grooves 74 communicated with the grooves 71 between adjacent two of the wells 3 may be formed outside of the most outside wells 3 of the plurality of wells 3, and the frame portion 2a of the plastic plate 2 may have relief grooves 72 open to the side faces 73a, 73b and 73c and grooves 74 of the plastic plate 2, so that the sample housed in the grooves 71 may escape to the outside of the plastic plate 2 via the grooves 74 and relief grooves 72. Alternatively, the groove 71 between adjacent two of the wells 3 may be open directly to the side faces 73a, 73b and 73c of the plastic plate 2.

As shown in FIGS. 22A and 22B, if a groove 71 having a substantially rectangular or substantially U-shaped cross section formed between adjacent two of the wells 3 has inclined surfaces 71a and 71a in the upper portions of both side walls facing each other, the sample dropping between the adjacent two of the wells 3 is guided on the inclined surfaces 71a and 71a to easily drop into the groove 71.

In this preferred embodiment, a treatment for giving a hydrophilic property (e.g., UV treatment or plasma treatment) is carried out on at least the surface of the groove 71 and the surface 7 between the wells 3. Thus, the sample dropping between adjacent two of the wells 3 can be surely acquired into the groove 71. In addition, the treatment for giving a hydrophilic property to the grooves 74 and relief groove 72 may be carried out.

In this preferred embodiment, since the groove 71 is formed between adjacent two of the wells 3 in order to prevent different kinds of samples from being mixed with each other, it is not always required to form the groove 71 between adjacent two of the wells 3 into which the same kind of samples are injected. In addition, when quantitative analysis or the like is carried out, the groove 71 is designed to prevent a sample, which has not been injected into one of the adjacent two of the wells 3, from entering the other well 3, and to ensure a predetermined amount of sample to be injected into the other well 3, so that it is not always required to form the groove 71 between adjacent two of the wells 3 wherein it is not required to suppose the displacement of the sample to be injected. Furthermore, while the opening edge 70 of each of the wells 3 has been surrounded by the grooves 71 in the embodiment shown in FIGS. 18B and 18E as an example, the present invention should not be limited thereto. For example, the groove 71 may be formed only between adjacent two of the wells 3 in X directions, or only between adjacent two of the wells 3 in Y directions.

The groove 71 formed between adjacent two of the wells 3 in this preferred embodiment may be formed between adjacent two of the wells 26 of the plastic plate 22 in the above described second preferred embodiment, or between adjacent two of the wells 61 of the plastic plate 60 in the above described sixth preferred embodiment. Moreover, the embodiment wherein the recess is formed around the opening edge 70 of each of the wells 3 as shown in FIG. 18C and 20 may be applied to a portion surrounding the opening edge of each of the wells 26 of the plastic plate 22 in the above described second preferred embodiment, or to a portion surrounding the opening edge of each of the wells 61 of the plastic plate 60 in the above described sixth preferred embodiment.

### [Eighth Preferred Embodiment]

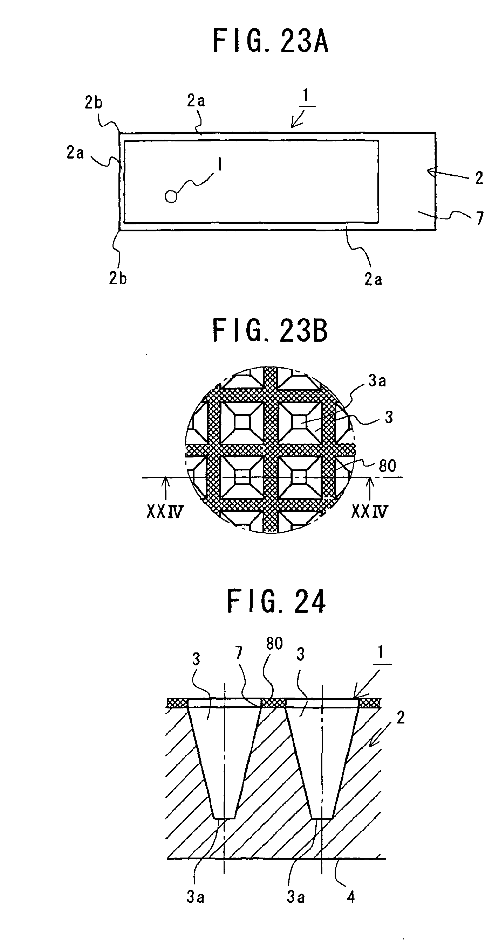

FIGS. 23A, 23B and 24 show the eighth preferred embodiment of a plastic plate 2 for a microarray 1 according to the present invention as an applied example of the plastic plate 2 in the first preferred embodiment. Furthermore, in this preferred embodiment, the same reference numbers as those in the plastic plate 2 in the above described first preferred embodiment are given to the same portions as those in the first preferred embodiment to omit repeated explanation.

As shown in these figures, a sample absorbing layer (e.g., a coating layer of a water absorbable polymer, a water absorbable porous material, a water absorbable raising material, or a water absorbable fiber layer) 80 capable of absorbing a sample is formed on the surface 7 of a plastic plate 2 between adjacent two of wells 3.

According to this preferred embodiment with such a construction, when a sample is injected into each of the wells 3, even if the position of the injected sample is displaced so that the sample drops into a space between adjacent two of the wells 3, the sample can be absorbed into the sample absorbing layer 80. As a result, when different kinds of samples are injected into adjacent two of the wells 3, respectively, the samples incorrectly injected into the wells 3 are absorbed into the sample absorbing layer 80. Thus, it is possible to prevent the samples from being erroneously injected into wells 3 other than wells 3 into which the samples are to be originally injected, and it is possible to prevent the different kinds of samples from being mixed with each other.

Furthermore, the sample absorbing layer 80 in this preferred embodiment may be formed on the surface 25 between adjacent two of the wells 26 of the plastic plate 22 in the above described second preferred embodiment, or on the surface 63 between adjacent two of the wells 61 of the plastic plate 60 in the above described sixth preferred embodiment.

If the sample absorbing layer 80 in this preferred embodiment has a light absorbing function, it is possible to prevent the crosstalk of light similar to the above described third preferred embodiment, so that it is possible to further improve the precision of measurement.

The sample absorbing layer 80 in this preferred embodiment is preferably applied on the surface 7 of the plastic plate 2 so as to be capable of being peeled off from the surface 7 of the plastic plate 2 after a sample is injected into each of the wells 3.

### [Other Preferred Embodiments]

While DNAs have been housed in the wells to be inspected in the above described first and second preferred embodiments, the present invention should not be limited thereto. The present invention may be applied to a vessel for housing therein and inspecting materials capable of being housed in wells to be analyzed, e.g., biopolymers, such as RNAs, amino acids, proteins and polysaccharides such as oligosaccharides, as well as to a vessel for housing therein and inspecting micro vital substances, such as viruses, or high molecular substances other than biopolymers (organic compounds), such as dioxin and environmental hormone, or inorganic substances, such as ultramicro harmful metals (metal compounds) contained in potable water or industrial waste water.

In the above described third preferred embodiment, the same light absorbable coating as the light absorbable coating 51 in the above described fourth preferred embodiment may be formed on the reverse 4 of the plastic plate 1 between adjacent two of the wells 3. Moreover, in the above described fourth preferred embodiment, the same light absorbable coating as the light absorbable coating 50 in the above described third preferred embodiment may be formed on the surface 7 of the plastic plate 2 between adjacent two of the wells 3.

As described above, according to the present invention, a sufficient amount of sample can be housed in the wells (recessed portions), so that it is possible to efficiently and more precisely inspect, analyze and house therein various samples.

According to the present invention, a sufficient amount of solution containing DNA fragments, specimens and reagents can be housed in the wells (recessed portions), so that it is possible to more precisely analyze genes.

According to the present invention, DNAs can be amplified in the wells (recessed portions), the plate can be used as a reactor in the PCR method. Thus, it is not required to provide any reactor in the PCR method which is required in the conventional microarray technique, and it is not required to apply DNA fragments on a glass plate from the reactor, so that it is possible to reduce the costs of equipment and operation time.

## Claims

1. A plastic plate capable of housing a plurality of samples to be analyzed, wherein a plurality of recessed portions for housing therein said samples are formed in a surface of the plastic plate by transferring a shape of a metal pattern.

2. A plastic plate as set forth in claim 1, wherein a heater housing portion is formed in a reverse of the plastic plate so as to correspond to said recessed portions.

3. A plastic plate as set forth in claim 1, wherein a heater housing portion is formed in a reverse of the plastic plate so as to correspond to said recessed portions, and a reinforcing rib is formed on the reverse of said plastic plate.

4. A plastic plate as set forth in any one of claims 1 through 3, wherein each of said recessed portions has a sectional shape taken in depth directions thereof, said sectional shape being a trapezoid so that an opening portion of each of said recessed portions has a larger area than that of a bottom thereof.

5. A plastic plate as set forth in any one of claims 1 through 3, wherein a large number of protrusions are formed on a bottom of each of said recessed portions.

6. A plastic plate as set forth in any one of claims 1 through 3, wherein said samples contain DNA fragments.

7. A plastic plate as set forth in any one of claims 1 through 3, which is made of a resin containing a light absorbable material.

8. A plastic plate as set forth in any one of claims 1 through 3, wherein a light absorbing coating is formed on said surface of the plastic plate between adjacent two of said recessed portions.

9. A plastic plate as set forth in any one of claims 1 through 3, wherein a light absorbable coating is formed on a reverse of the plastic plate so as to separate said recessed portions from each other.

10. A plastic plate as set forth in claim 1, wherein each of said recessed portions has a depth which is smaller than a width thereof.

11. A plastic plate as set forth in claim 1, wherein a groove is formed in said surface of the plastic plate between adjacent two of said plurality of recessed portions.

12. A plastic plate as set forth in claim 1, wherein a groove surrounding each of said recessed portions is formed in said surface of the plastic plate.

13. A plastic plate as set forth in claim 1, wherein an opening edge of each of said recessed portions is formed so as to protrude from said surface of the plastic plate.

14. A plastic plate as set forth in claim 1, wherein a sample absorbing layer surrounding an opening edge of each of said recessed portions is formed on said surface of the plastic plate.

15. A plastic plate assembly comprising:
a plurality of plastic plates capable of housing a plurality of samples to be analyzed; and
a frame having a plurality of plate holding holes for housing and holding therein said plurality of plastic plates,
wherein a plurality of recessed portions for housing therein said samples are formed in a surface of each of said plastic plates by transferring a shape of a metal pattern.

16. A plastic plate assembly as set forth in claim 15, wherein two pairs of side faces of each of said plastic plates are supported on side faces of a corresponding one of said plate holding holes at three points, respectively.

17. A plastic plate assembly as set forth in claim 15 or 16, wherein each of said plastic plates has a flange portion which has a bottom surface supported on an opening edge of a corresponding one of said plate holding holes at three points.

18. A plastic plate assembly as set forth in claim 15 or 16, wherein an engaging protrusion is formed on one of one side face of each of said plastic plates and one side face of a corresponding one of said plate holding holes facing the one side face of each of said plastic plates, and an engaged recessed portion to be engaged with said engaging protrusion is formed in the other of the one side face of each of said plastic plates and the one side face of the corresponding one of said plate holding holes.

19. A plastic plate assembly as set forth in claim 15 or 16, wherein a heater housing portion is formed in a reverse of each of said plastic plates so as to correspond to said recessed portions.

20. A plastic plate assembly as set forth in claim 15 or 16, wherein each of said recessed portions has a sectional shape taken in depth directions thereof, said sectional shape being a trapezoid so that an opening portion of each of said recessed portions has a larger area than that of a bottom thereof.

21. A plastic plate assembly as set forth in claim 15 or 16, wherein a large number of protrusions are formed on a bottom of each of said recessed portions.

22. A plastic plate assembly as set forth in claim 15 or 16, wherein each of said plastic plates has positioning means serving as a reference position when said samples are injected into said recessed portions.

23. A plastic plate assembly as set forth in claim 15 or 16, wherein said samples contain DNA fragments.

24. A plastic plate assembly as set forth in claim 15 or 16, wherein each of said plastic plates is made of a resin containing a light absorbable material.

25. A plastic plate assembly as set forth in claim 15 or 16, wherein a light absorbing coating is formed on said surface of each of said plastic plates between adjacent two of said recessed portions.

26. A plastic plate assembly as set forth in claim 15 or 16, wherein a light absorbable coating is formed on a reverse of each of said plastic plates so as to separate said recessed portions from each other.

27. A plastic plate assembly as set forth in claim 15, wherein each of said recessed portions of each of said plastic plates has a depth which is smaller than a width thereof.

28. A plastic plate assembly as set forth in claim 15, wherein a groove is formed in said surface each of said plastic plates between adjacent two of said plurality of recessed portions.

29. A plastic plate assembly as set forth in claim 15, wherein a groove surrounding each of said recessed portions is formed in said surface of each of saidplastic plates.

30. A plastic plate assembly as set forth in claim 15, wherein an opening edge of each of said recessed portions is formed so as to protrude from said surface of each of said plastic plates.

31. A plastic plate assembly as set forth in claim 15 wherein a sample absorbing layer surrounding an opening edge of each of said recessed portions is formed on said surface of each of said plastic plates.
